Europäisches Patentamt

European Patent Office

Office européen des brevets

(11) Veröffentlichungsnummer: **0 035 138**
**B1**

(12) **EUROPÄISCHE PATENTSCHRIFT**

(45) Veröffentlichungstag der Patentschrift: **30.01.85**

(21) Anmeldenummer: **81100944.8**

(22) Anmeldetag: **11.02.81**

(51) Int. Cl.⁴: **H 03 B 25/00,** A 61 N 1/32,
H 03 B 5/24

(54) Schaltungsanordnung zur Erzeugung eines niederfrequenten Wechselstromes für die Elektrotherapie.

(30) Priorität: **29.02.80 DE 3007717**

(43) Veröffentlichungstag der Anmeldung:
**09.09.81 Patentblatt 81/36**

(45) Bekanntmachung des Hinweises auf die
Patenterteilung:
**30.01.85 Patentblatt 85/05**

(84) Benannte Vertragsstaaten:
**AT CH FR GB IT LI**

(56) Entgegenhaltungen:
**DE-A-2 023 716**
**DE-A-2 545 675**
**DE-A-2 745 349**
**GB-A- 804 472**
**GB-A-1 063 871**
**US-A-3 056 409**
**US-A-4 180 079**

(73) Patentinhaber: **Scherer, Erich**
**Wiesenstrasse 18**
**D-6603 Sulzbach (DE)**

(72) Erfinder: **Scherer, Erich**
**Wiesenstrasse 18**
**D-6603 Sulzbach (DE)**

(74) Vertreter: **Holzhäuser, Peter Karl, Dr.-Ing. et al**
**Patentanwälte Dr.-Ing. P.K. Holzhäuser Dipl.-**
**Met. W. Goldbach Dipl.-Ing.Ing. L.**
**Schieferdecker Herrnstrasse 37**
**D-6050 Offenbach am Main (DE)**

(56) Entgegenhaltungen:
QST FOR, Jahrgang 47, nr. 7, Juli 1963
NEWINGTON (US) M.I. NEIDICH "Two-tone test
oscillator using transistors", Seiten 20, 21
FUNKSCHAU Jahrgang 42, nr. 20, Oktober
1970 MÜNCHEN (DE) G. ZIMMERMANN
"Transistor-RC-Oszillator zum Steuern von
Horizontal-Endstufen", Seiten 705-707

EP 0 035 138 B1

## Beschreibung

Die Erfindung bezieht sich auf eine Schaltungsanordnung für die Elektrotherapie, bei der mehr als zwei Sinusgeneratoren je eine Grundfrequenz von etwa 4000 Hz sowie niederfrequente Schwebungen als Therapieströme im Bereich von 0 bis 100 Hz erzeugen, die überlagert werden.

Bei einem bekannten Elektrotherapie-Gerät dieser Art (DE—C—25 45 675) sind drei Sinusgeneratoren vorgesehen, die Wechselströme im Bereich von 1 bis 100 kHz erzeugen, wobei sich die Frequenzen der drei Wechselströme um 0—200 Hz unterscheiden. Die sechs Ausgänge dieser Sinusgeneratoren sind direkt an je eine von sechs Elektroden angelegt, wobei die feste relative Anordnung der Elektroden so getroffen ist, daß sich jeweils die beiden zu dem gleichen Sinusgenerator gehörenden Elektroden in der Anordnung etwa diametral gegenüberliegen. Damit soll erreicht werden, daß sich die drei Wechselströme als Therapieströme innerhalb des Körpers in dem von den Elektroden umgrenzten Bereich überlagern, wobei sie infolge der unterschiedlichen Frequenzen der drei Wechselströme zur Interferenz kommen sollen.

Elektrotherapiegeräte dieser Art werden als sogenannte "Interferenzstrom-Therapiegeräte" bezeichnet.

Diese Geräte besitzen den Nachteil, daß wegen der gewünschten Tiefenwirkung der "Interferenzimpulse" mit Strömen von mehr als 10 mA pro Generatorausgang gearbeitet werden muß und daß die "Interferenzbildung" im Körper abhängig von der Homogenität oder Inhomogenität der jeweiligen Körperzone ist.

Der Erfindung liegt die Aufgabe zugrunde, ein Elektrotherapiegerät der eingangs genannten Art so auszugestalten, daß an nur einem zu verwendendem Elektrodenpaar ein Therapiestrom anliegt, der eine Grundfrequenz von etwa 4000 Hz, ein breites Oberwellenband und im Bereich von 1—100 Hz an- und abschwellende niederfrequente Schwingungen führt, wobei möglichst keine taktmäßig auftretenden niederfrequenten Schwingungen auftreten sollen, so daß der natürliche Vorgang der Handmassage möglichst gut nachgeahmt wird.

Diese Aufgabe wird erfindungsgemäß dadurch gelöst, daß jeder der Sinusgeneratoren einen Ausgangskreis enthält, der ein Potentiometer aufweist, dem die Primärseite eines Übertragers parallel gelegt ist, wobei die Sekundärseite des Übertragers parallel mit den sekundärseiten der Übertrager der übrigen Sinusgeneratoren an einen gemeinsamen Ausgang gelegt ist, wodurch zeitliche Änderungen der Amplituden der Sinusgeneratoren als niederfrequente Schwebungen erzeugt werden, die instabil zwischen 0 und 100 Hz schwanken und der gemeinsame Ausgang an den Eingang eines Niederfrequenzverstärkers gelegt ist, dessen Ausgang den Therapiestrom liefert.

Es ist an sich bekannt, die Ausgangsspannungen mehrerer Generatoren durch Zusammenschalten zu summieren. Nach den Erfindungsmerkmalen sind demnach die Schwingungen der drei Generatoren unterschiedlich und variieren in unterschiedlichen Bereichen, wobei dies auch für die Oberwellen jedes Generators gilt. Durch die Addition dieser Schwingungen in den zusammengefaßten Ausgängen der Übertrager ergeben sich nun zeitliche Änderungen der Amplituden der Schwingungen jedes der Generatoren. Aus dem Zusammenwirken aller Maßnahmen ergibt sich so der gewünschte instabile Frequenz- und Amplitudenverlauf an den beiden Elektroden.

Vorteilhafte Ausgestaltungen der Erfindung ergeben sich aus den Unteransprüchen.

Die Erfindung ist im folgenden anhand der Zeichnungen beispielsweise näher erläutert, und zwar zeigt:

Fig. 1: ein Schaltbild eines bevorzugten Ausführungsbeispiels,

Fig. 2: rein schematisch die Ausgänge der einzelnen Generatoren,

Fig. 2a: ein Foto des entsprechenden Oszillographenbildes,

Fig. 3: die Hüllkurven der unmodulierten Ausgangsspannung,

Fig. 4: die zeitlich aufeinanderfolgende Änderung der Hüllkurven, nach entsprechender Modulation, folgt ursprüngliche Beschreibungsseite 4

Fig. 5 eine auseinandergezogene Darstellung zur Erläuterung der Fig. 4,

Fig. 6 eine mechanische, automatische Regelvorrichtung für die Potentiometer

Fig. 7 eine Schaltungsanordnung nach einer Ausgestellung der Erfindung und

Fig. 8 rein schematisch den Ausgang der Schaltungsanordnung nach Fig. 7,

Fig. 8a ein Foto des entsprechenden Oszillographenbildes.

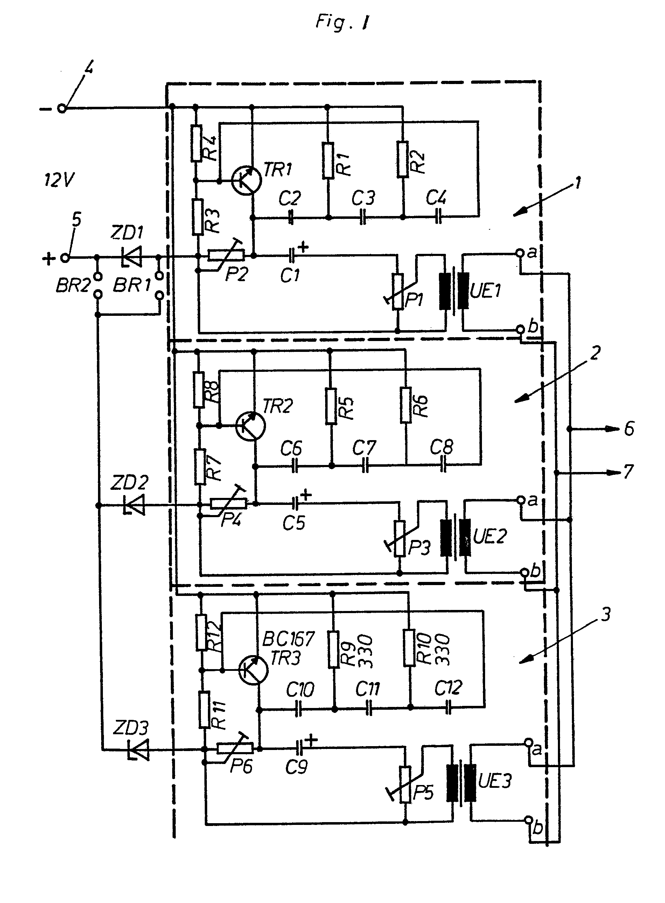

Das Schaltbild nach Fig. 1 zeigt drei Wechselstromgeneratoren 1, 2 und 3, die jeweils über Zenerdioden ZD 1 bis ZD 3 an die Eingangsklemmen 4 und 5 gelegt sind. An den Eingangsklemmen liegt beispielsweise eine Eingangsspannung von 12 Volt an.

Über einen Spannungsteiler R 3 bis R 4 wird ein Transistor TR 1 angesteuert, dessen Arbeitspunkt über ein Potentiometer P 2 steuerbar ist. An die Basis und den Kollektor des Transistors ist ein Schwingkreis gelegt, der aus einem Widerstand R 1 und einem Kondensator C 2 besteht. Parallel zu diesem ersten Schwingkreis liegt ein zweiter Schwingkreis mit dem Widerstand R 2 und dem Kondensator C 3. An diesen zweiten Schwingkreis ist ein Kondensator C 4 angeschlossen, der andererseits an die Basis des Transistors TR 1 gelegt ist.

Infolge unterschiedlicher Dimensionierung der Elemente des ersten und des zweiten Schwingkreises werden Oberwellen erzeugt und der Rückkopplungskreis mit dem Kondensator C 4 erzeugt eine Modulation der erzeugten Schwingung, die an den Ausgangskreis gelangt. Dieser Ausgangskreis umfaßt
außer dem schon genannten Potentiometer P 2
einen Kondensator C 1 und ein Potentiometer P
1. Parallel zu dem Potentiometer P 1 ist ein
Übertrager UE 1 an die Ausgangsleitungen 6
und 7 gelegt.

Die Generatoren 2 und 3 entsprechen dem
grundsätzlichen Aufbau des Generators 1,
besitzen jedoch wiederum anders dimensionierte Elemente.

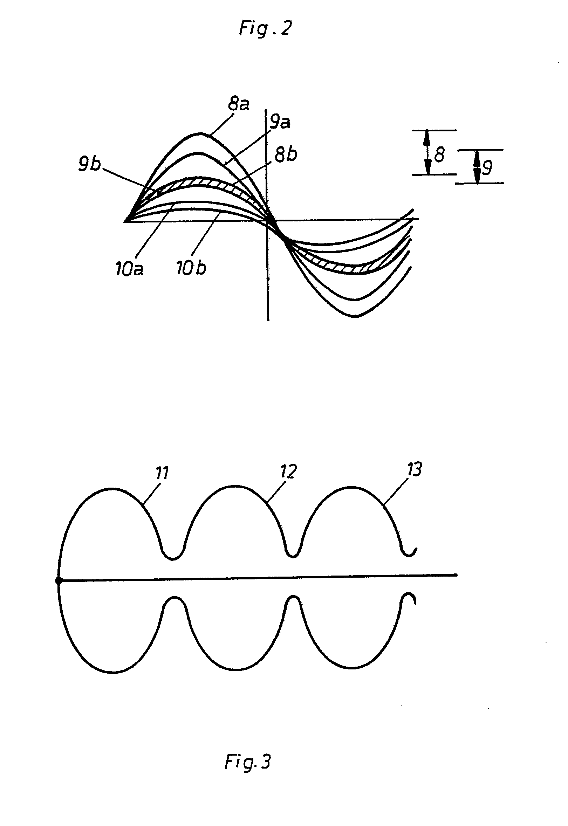

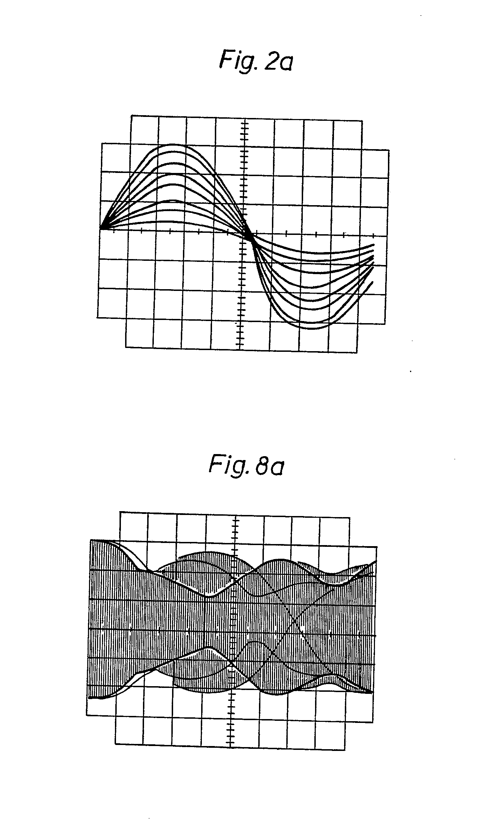

Die Ausgänge der drei Generatoren sind
anhand der Fig. 2 rein schematisch erläutert. So
liefert z.B. der Generator 1 eine Anzahl von
Schwingungen, die zwischen den Hüllkurven 8a
und 8b liegen. Der Generator 2 liefert Schwingungen, die zwischen den Hüllkurven 9a und 9b
liegen, während die Schwingungen des
Generators C zwischen den Hüllkurven 10a und
10b liegen.

Es ist ersichtlich, daß die Frequenzen der
Schwingungen der Generatoren unterschiedlich sind und auch die von einem einzigen
Generator erzeugten Schwingungen in einem
gewissen Frequenzbereich variieren. Durch die
Addition der Schwingungen in den zusammengefaßten Ausgängen der Generatoren ergibt
sich nun eine zeitliche Änderung der
Amplituden der Schwingungen des Generators
1 in dem in Fig. 2 mit 8 angedeuteten Bereich,
während die Amplituden der Schwingungen des
Generators 2 in dem mit 9 bezeichneten
Bereich variieren.

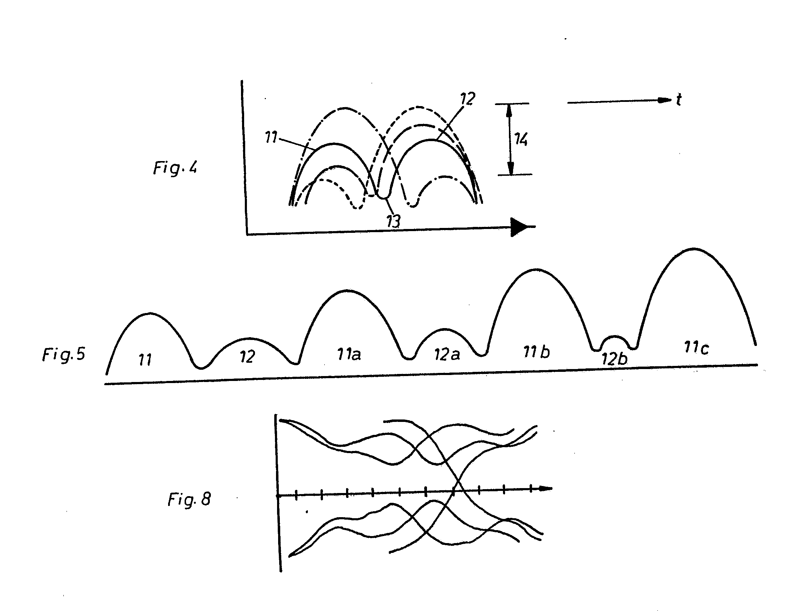

Rein sumarisch ergeben sich somit über eine
Grundfrequenz von 4000 Hz. Schwingungen,
deren Hüllkurve in Fig. 3 im statischen Zustand
mit 11 bezeichnet ist.

Durch geeignete Einstellung der Potentiometer P1, P3 und P5 ergeben sich zeitliche
Änderungen der Amplituden der Schwingungen, die als Schwebungen gedeutet werden
können. Wie aus Fig. 4 ersichtlich, ändern sich
die benachbarten Schwingungsbäuche 11 und
12 der Darstellung nach Fig. 3 in zeitlicher
Folge etwa derart, daß z.B. zunächst der
Schwingungsbauch 11 seine Amplitude verringert, so daß aus der Darstellung nach der
ausgezogenen Linie die Kurvenform nach der
unterbrochenen Linie und danach die nach der
punktierten Linie wird. Gleichzeitig verlagert
sich der Schwingungsknoten 13. Nach
Erreichen eines Minimums wächst dann die
Amplitude des Schwingungsbauches 11 wieder
an, während die Amplitude des Schwingungsbauches 12 abnimmt, bis die in strichpunktierter Linie dargestellte zweite Endlage
erreicht ist. Diese Amplitudenvariation ist in Fig.
4 mit 14 bezeichnet.

Fig. 5 zeigt nun beispielsweise einige aufeinander folgende Oszillografen-Bilder der
Schwingungsbäuche 11 und 12, die hier der
Übersichtlichkeit halber nebeneinander zusammengefügt sind.

Die Amplituden der Hüllkurven 11, 12
werden somit durch Schwebungen moduliert,
wobei diese Modulationsfrequenz im Bereich
von 0 bis 100 Hz. variiert werden können. Die
dargestellten Amplituden entsprechen in ihrem
Maximum einer Spannung von 12 Volt und
können durch einen an den Ausgang 15 der
gesamten Schaltungsanordnung gelegten
Regelwiderstand auf einen Bereich von 2 bis 12
Volt eingestellt werden.

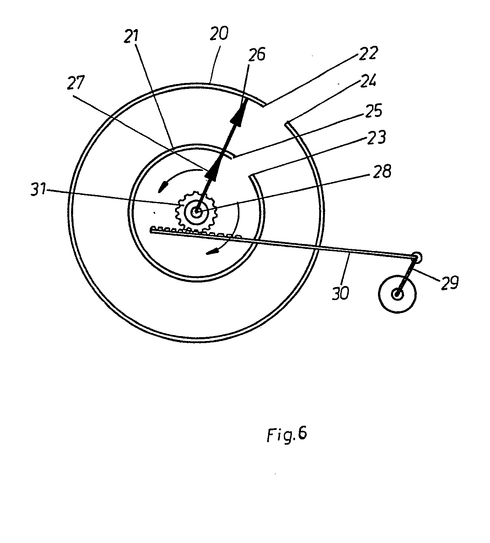

Es ist jedoch auch möglich, die Änderungsfrequenz und/oder die Amplitude der Schwingungen automatisch zu steuern. Ein Ausführungsbeispiel hierzu ist rein schematisch in Fig. 6
dargestellt.

Fig. 6 ziegt einen Drehwiderstand mit zwei
Widerstandsbahnen 20 und 21. Der Eingang
der Widerstandsbahn 20 ist mit 22 bezeichnet
und der Eingang der Widerstandsbahn 21 mit
23. Der Ausgang der Widerstandsbahn 20 ist
mit 24 und der Ausgang der Widerstandsbahn
21 mit 25 bezeichnet.

Es ist nun ersichtlich, daß beim Drehen der
Schleifer 26 und 27, die an einen gemeinsamen Auschluß 28 gelegt sind, in der einen
Richtung der Widerstand auf der äußeren Bahn
zunimmt, während gleichzeitig der Widerstand
der inneren Bahn abnimmt.

Um die Bewegung der Schleifer in der einen
bzw. der anderen Richtung vollautomatisch
ablaufen zu lassen, kann beispielsweise ein
Kurbelantreib 29 vorgesehen sein, an dessen
Kurbel eine Zahnstange 30 angelenkt ist, die
auf ein Antriebsritzel 31 einwirkt.

Aus der DE—OS 25 45 675 ist eine Vorrichtung zur Erzeugung von Wechselströmen für
die Elektro-Therapie bekannt, bei der drei
Generatoren je einen Wechselstrom erzeugen,
der an je ein Elektrodenpaar gelegt wird. Dabei
erzeugt beispielsweise einer der Generatoren
eine Frequenz von 4000 Hz., der zweite
Generator eine Frequenz zwischen 4050 und
4100 Hz., während der dritte Generator eine
Frequenz erzeugt, die von 4000 Hz. nur um
einen ganz geringen Betrag abweicht, Die an
den Körper gelegten sechs Elektroden erzeugen
zwischen sich ein elektro-magnetisches Feld,
welches Interferenzen bilden soll.

Andere bekannte Generatoren zur Erzeugung von Strömen für die Elektrotherapie sind so
ausgebildet, daß einzelne Impulse erzeugt
werden, die im wesentlichen Dreieckform
besitzen, die ferner eine kurve Impulsdauer und
im Verhältnis dazu relativ große Impulsabstände besitzen. Dabei ist die Impulsfrequenz genau einstellbar.

Hierzu wird beispielsweise auf das Buch von
Steuernagel aus der Reihe "Skripten zur
Elektrotherapie" verwiesen.

Diese bekannten Generatoren arbeiten, wie
bereits oben erwähnt wurde, eingangsseitig mit
der Netzfrequenz und enthalten Ausgangsübertrager. Infolge der verwendeten Netzfrequenz
können durch die induktive Übertragung Spannungsspitzen bzw. Stromspitzen erzeugt
werden, deren Werte für die elektrotherapeutische Behandlung gefährlich werden können.

Außerdem sind die von den bekannten Generatoren erzeugten Impulse reine Spitzenimpulse, deren Wirkung derjenigen einer Stromstoß-Therapie entspricht.

Im Gegensatz hierzu wird mit der oben beschriebenen Erfindung als Ausgang der Generatoranordnung ein Spannungsverlauf erzielt, bei dem die ursprüngliche Sinusform stets erhalten bleibt. Es treten keine Spannungs- bzw. Stromspitzen auf.

Dies wird hauptsächlich durch den hohen Anteil von Oberwellen in den Ausgangssignalen der einzelnen Generatoren bewirkt.

Dementsprechend entstehen auch keine fest einstellbaren Ausgangsfrequenzen, sondern immer nur einstellbare Ausgangs-Frequenzbereiche, also z.B. von 5 bis 20 Hz., bei denen lediglich der Mittelwert einigermaßen konstant ist.

Durch Stellen der Eingangspotis der Generatoren (P2, P4, P6) können die Schwingungsfrequenzen jeweils bis zu 300 Hz. verschoben werden. Durch die Überlagerung der Ausgänge von drei Generatoren ergeben sich so Schwebungen, deren Frequenzbereich von 0 bis 100 Hz. verstellbar sind und die die definierten Ausgangsimpulse der bekannten Generatoren ersetzen.

Für ein bevorzugtes Ausführungsbeispiel der Erfindung ist ein Bestückungsplan mit der Bemessung der einzelnen Bauelemente beigefügt.

Mit dieser Bestückung der Schaltungsanordnung nach Fig. 1 wurden die in den Figuren 2—5 dargestellten Kurven erhalten.

Das entscheidende Merkmal des Strom- bzw. Spannungsverlaufes nach der Erfindung besteht darin, daß die Amplituden sich fortwährend ändern und daß die Schwingungsknoten 13 nahe der Null-Linie liegen. Durch diese Merkmale wird nach jedem von der behandelten Körperstelle aufgenommenen Schwingungsknoten eine Entlastung der Körperzellen erreicht. Es wird auf diese Weise gleichsam die Massage von Hand nachgeahmt.

Dabei bleibt jedoch die Grundfrequenz des ausgehenden Stromes von 4.000 Hz. grundsätzlich erhalten, lediglich die durch die Hüllkurven definierten Frequenzen, die zugleich mit der Schwebungsfrequenz variieren, bilden mit ihrer relativ geringen und dazu einstellbaren Intensität den wirksamen Anteil des auf den Körper einwirkenden Stromes.

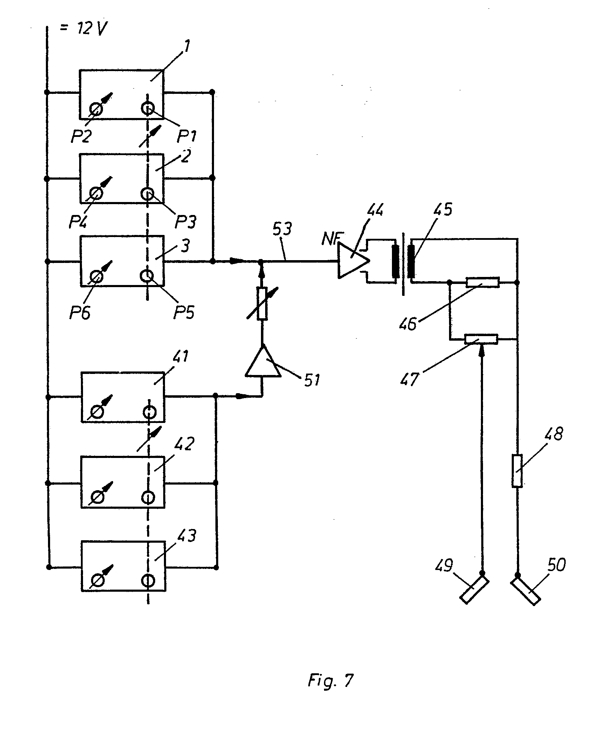

Ein besonders vorteilhafte weitere Ausgestaltung der Erfindung ist in Fig. 7 in Form eines Blockschaltbildes dargestellt.

In Fig. 7 entsprechen die drei Generatoren 1, 2 und 3 denen nach Fig. 1. Die einstellbaren Potentiometer P2, P4 und P6 sind dabei noch einmal gesondert dargestellt. Die Potentiometer P1, P3 und P5 sind mittels einer Anordnung nach Fig. 6 miteinander gekoppelt.

Diesen drei Generatoren ist eine Gruppe von drei Generatoren 41, 42 und 43 parallel an den Eingang angeschlossen. Diese letztgenannten Generatoren entsprechen in ihrem Aufbau vollständig denen nach Fig. 1.

Die Ausgänge der ersten Gruppe von Generatoren 1, 2, 3 sind an einen gemeinsamen Ausgang 53 gelegt. Dieser speist einen Niederfrequenzverstärker 44, dessen Ausgang an einen Übertrager 45 gelegt ist. Die Sekundärseite des Übertragers 45 enthält einen Lastwiderstand 46 und ein Potentiometer zum Einstellen der Ausgangsspannung. Außerdem enthält der Ausgang noch einen Lastwiderstand 48. Die beiden Ausgangsleitungen sind an an sich bekannte Elektroden 49 und 50 gelegt.

Gemäß der Weiterbildung der Erfindung sind die Generatoren 41, 42 und 43 an einen Vorverstärker 51 gelegt, der zugleich der Entkopplung dient. Er verhindert, daß der Ausgang der sich gegenseitig beeinflussenden Generatoren 1, 2 und 3 auch die Generatoren 41, 42 und 43 beeinflussen kann.

Der Ausgang des Vorverstärkers 51 ist über einen regelbaren Widerstand 52 an die Ausgangsleitung 53 der ersten Generatorengruppe gelegt.

Die Hüllkurve des Ausgangssignals an den Elektroden 49 und 50 ist rein schematisch in Fig. 8 in einem Beispiel dargestellt. Diese Darstellung entspricht im wesentlichen derjenigen nach Fig. 4, zeigt also einen zeitlichen Ausschnitt aus dem Strom- bzw. Spannungsverlauf. In der Darstellung nach Fig. 8 entspricht der langwelligste Anteil der Schwebungskurve einer Frequenz von etwa 100 Hz., während der kurzwelligste Anteil einer Frequenz von etwa 12 Hz. entspricht. Die Schwebungsfrequenz selbst liegt dabei etwas unterhalb von 100 Hz.

Aus der Darstellung nach Fig. 8 ist gut die Überlagerung der Ausgänge der beiden Generatorengruppen zu ersehen, die im Kurvenverlauf zu weiteren Amplituden-Modulationen in dem kurzwelligeren Bereich führt.

Es darf hier nochmals darauf hingewiesen werden, daß diese Hüllkurve wie auch die Hüllkurve nach den Figuren 3—5 den mittelfrequentigen Anteil von Schwingungen von 4000 Hz. enthalten.

Für die praktische Ausführung einer erfindungsgemäßen Schaltungsvorrichtung sei hier erwähnt, daß die Potentiometer P2, P4 und P6 und die entsprechenden Potentiometer der zweiten Generatorengruppe anhand von Tabellenwerten eingestellt werden können und daß die Einstellung der Widerstände 52 und 47 nach den jeweiligen Bedürfnissen und der Verträglichkeit des Patienten eingestellt werden.

Bestückung der Platine

| | |
|---|---|
| R1=470 Ω | C1=4,7 $\mu$F |
| R2=470 Ω | C2=33 n |
| R3=33 kΩ | C3=33 n |
| R4=6,8 kΩ | C4=22 n |
| R5=390 Ω | C5=4,7 $\mu$ |
| R6=390 Ω | C6=33 n |
| R7=33 kΩ | C7=33 n |
| R8=6,8 kΩ | C8=33 n |
| R9=330 Ω | C9=4,7 $\mu$ |
| R10=330 Ω | C10=33 n |
| R11=33 kΩ | C11=33 n |
| R12=6,8 kΩ | C12=47 n |

P1=10 kΩ
P2=1 kΩ
P3=10 kΩ
P4=1 kΩ
P5=10 kΩ
P6=1 kΩ

ZD1 bis 3=6 V
UE 1—3=St 855
TR 1—3=BC 167

## Patentansprüche

1. Schaltungsanordnung für die Elektrotherapie, bei der mehr als zwei Sinusgeneratoren (1—3) je eine Grundfrequenz von etwa 4000 Hz sowie niederfrequente Schwebungen als Therapieströme im Bereich von 0 bis 100 Hz erzeugen, die überlagert werden, dadurch gekennzeichnet, daß jeder der Sinusgeneratoren (1) einen Ausgangskreis ($P_1$, $C_1$ $P_2$) enthält, der ein Potentiometer ($P_1$) aufweist, dem die Primärseite eines Übertragers ($UE_1$) parallel gelegt ist, wobei die Sekundärseite (a, b) des Übertragers parallel mit den Sekundärseiten (a, b) der Übertrager ($UE_2$, $UE_3$) der übrigen Sinusgeneratoren (2, 3) an einen gemeinsamen Ausgang (6, 7) gelegt ist, wodurch zeitliche Änderungen der Amplituden der Sinusgeneratoren (1—3) als niederfrequente Schwebungen erzeugt werden, die instabil zwischen 0 und 100 Hz schwanken und der gemeinsame Ausgang (6, 7) an den Eingang eines Niederfrequenzverstärkers (44) gelegt ist, dessen Ausgang den Therapiestrom liefert.

2. Schaltungsanordnung nach Anspruch 1, dadurch gekennzeichnet, daß jeder Generator ein weiteres Potentiometer ($P_2$) zur Steuerung des Arbeitspunktes eines Transistors enthält.

3. Schaltungsanordnung nach Anspruch 2, dadurch gekennzeichnet, daß das weitere Potentiometer ($P_2$) auch Bestandteil des Ausgangskreises ist.

4. Schaltungsanordnung nach einem der vorhergehenden Ansprüche, dadurch gekennzeichnet, daß der Ausgang des Niederfrequenz-Verstärkers (44) über einen Übertrager (45) an die beiden Elektroden (49, 50) gelegt ist.

5. Schaltungsanordnung nach Anspruch 4, dadurch gekennzeichnet, daß der Übertrager (45) über ein Potentiometer (47) und einen Widerstand (48) an die Elektroden angeschlossen ist und parallel zu ihm ein Lastwiderstand (46) gelegt ist.

6. Schaltungsanordnung nach Anspruch 5, dadurch gekennzeichnet, daß das Potentiometer (47) mit einer Vorrichtung (29, 30, 31) zur vollautomatischen Verstellung versehen ist.

## Revendications

1. Circuit pour l'électrothérapie dans lequel plus de deux générateurs d'ondes sinusoïdales (1—3) produisent chacun une fréquence de base d'environ 4000 Hz ainsi que des battements de basse fréquence en tant que courants thérapeutiques dans la zone de 0 à 100 Hz, ces courants étant superposés, caractérisé en ce que chaque générateur d'ondes sinusoïdales (1) comporte un circuit de sortie ($P_1$, $C_1$, $P_2$) qui présente un potentiomètre ($P_1$) commuté en parallèle avec le côté primaire d'un transformateur ($UE_1$), le côté secondaire (a, b) du transformateur étant commuté en parallèle avec le côté secondaire (a, b) des transformateurs ($UE_2$, $UE_3$) des autres générateurs d'ondes sinusoïdales (2, 3) à une sortie commune (6, 7), ce qui produit des modifications dans le temps des amplitudes des générateurs d'ondes sinusoïdales (1—3) en tant que battements à basse fréquence variant de façon instable entre 0 et 100 Hz, et que la sortie commune (6, 7) est appliquée à l'entrée d'un amplificateur à basse fréquence (44) dont la sortie produit le courant thérapeutique.

2. Circuit selon la revendication 1, caractérisé en ce que chaque générateur comporte un autre potentiomètre ($P_2$) pour commander le point de fonctionnement dynamique d'un transistor.

3. Circuit selon la revendication 1, caractérisé en ce que l'autre potentiomètre ($P_2$) est aussi un élément du circuit de sortie.

4. Circuit selon l'une des revendications précédentes, caractérisé en ce que la sortie de l'amplificateur à basse fréquence (44) est appliquée aux deux électrodes (49, 50) par un transformateur (45).

5. Circuit selon la revendication 4, caractérisé en ce que le transformateur (45) est relié aux électrodes par un potentiomètre (47) et une résistance (48) et qu'une résistance de charge (46) est commutée en parallèle au transformateur.

6. Circuit selon la revendication 5, caractérisé en ce que le potentiomètre (47) est muni d'un dispositif (29, 30, 31) pour un réglage complètement automatique.

## Claims

1. A circuit system for electrotherapy in which more than two sine wave generators (1—3) each produce a base frequency of about 4000 Hz and low frequency beat oscillations as therapeutic currents in a range of 0 to 100 Hz, same being superimposed, characterized in that each of the sine wave generators (1) has an output circuit ($P_1$, $C_1$ $P_2$), that has a potentiometer ($P_1$), that is joined in parallel with the primary side of a transformer ($UE_1$), and the secondary side (a, b) of the transformer in parallel with the secondary sides (a, b) of the transformers ($UE_2$, $UE_3$) of the other sine wave generators (2, 3) is joined with a common output (6, 7) so that changes in time of the amplitudes of the sine wave generators (1 to 3) are produced as low frequency beats varying instably between 0 and 100 Hz, and the common output (6, 7) is joined with the input of a low frequency amplifier (44), whose output supplies the therapeutic current.

2. The circuit system as claimed in claim 1 characterized in that each generator has a further potentiometer ($P_2$) for control of the operating point of a transistor.

3. The circuit system as claimed in claim 2 characterized in that the further potentiometer ($P_2$) is as well a part of the output circuit.

4. The circuit system as claimed in any one of the claims hereinbefore characterized in that the output of the low frequency amplifier (44) is joined up with the two electrodes (49, 50) by way of transformer (45).

5. The circuit system as claimed in claim 4 characterized in that the transformer (45) is joined up with the electrodes by way of a potentiometer (47) and a resistor (48) and parallel thereto there is a load resistor (46).

6. The circuit system as claimed in claim 5 characterized in that the potentiometer (47) has a means (29, 30, 31) for fully automatic adjustment.

O 035 138

*Fig. I*

Fig . 2

Fig. 3

*Fig. 2a*

*Fig. 8a*

Fig. 4

Fig. 5

Fig. 8

0035138

3

Fig.6

4

Fig. 7